# EUROPEAN PATENT SPECIFICATION

(11) **EP 3 316 843 B1**
(45) Date of publication and mention of the grant of the patent: **18.12.2019**
(21) Application number: 16747549.0
(22) Date of filing: 30.06.2016
(51) Int. Cl.: A61G 9/00, B65D 5/40

(54) **LIQUID CONTAINER**
FLÜSSIGKEITSBEHÄLTER
RÉCIPIENT POUR LIQUIDE

(30) Priority: 30.06.2015 GB 201511435
(43) Date of publication of application: 09.05.2018
(73) Proprietor: P-Pot Ltd, Derby, Derbyshire DE1 1NG (GB)
(72) Inventor: WELLS, Alison, Sutton Scotney Hampshire SO21 3PT (GB); PIPER, Martin, Sutton Scotney Hampshire SO21 3PT (GB); CAULCUTT, John, Sutton Scotney Hampshire SO21 3PT (GB); DAVIES, Giles, Derby, Derbyshire DE1 1NG (GB)
(74) Representative: Butler, Kathryn Louise
(86) International application number: PCT/GB2016/051963
(87) International publication number: WO 2017/001852

(56) References cited:
- WO-A1-98/01350
- GB-A- 1 019 386
- US-A- 3 099 017
- US-A- 3 535 714
- US-A- 3 746 240

## Description

The present invention relates to liquid containers, and particularly although not exclusively, to collapsible containers configured to receive liquid. More specifically, the invention relates to a collapsible container configured for use as a male urine bottle.

In the healthcare sector, disposable male urine bottles are used for male patients who are bed-bound. The device which is currently used is made from recycled wood/paper-based pulp, and is a rigid and awkward shape, which means it takes up a lot of valuable hospital space to store. In addition, transport and logistics costs are high due to the large volume of the device.

US5065449 attempts to provide a solution to this problem by providing a device comprising a collapsible cylindrical collar and a plastic membrane. However, the membrane makes the device expensive to manufacture, and means that this device does not comply with the macerator disposal system installed in most hospital facilities, thereby making disposal difficult. Additionally, this device is unable to sit on a flat surface holding urine for up to 5 hours without leaking.

US3746240 discloses a collapsible, disposable urine container formed from a unitary blank having hingedly connected walls adapted to form an open ended enclosure. Bottom panels hingedly connected to the walls close one end of the enclosure and automatically lock together to maintain the container in the erected state. A flexible, liquid impervious bag is mounted for reception in the enclosure formed by the walls and is secured to the walls. The walls are scored adjacent the open end of the enclosure to facilitate sealing of the bags.

The present disclosure arises from the inventors' work in trying to overcome the problems associated with prior art disposable male urine bottles.

In accordance with the invention, there is provided a liquid container comprising a rigid planar material defining first and second faces interconnected by at least two connecting edges, each face having an inner surface and an outer surface, wherein the container is configured to be converted, upon application of substantially opposing forces to two sides of the container, from a planar configuration in which the inner surface of the first face is substantially adjacent to the inner surface of the second face, to an open configuration in which the inner surfaces of the first and second faces are spaced apart and which, in combination with the at least two connecting edges, define a volume therebetween for containing liquid, and wherein the container comprises locking means, which is configured to hold the container in the open configuration, wherein the locking means comprises a tab disposed in the first face, and a first aperture disposed in the second face, wherein the first aperture is configured to receive the tab, thereby holding the container in the open configuration, and characterised in that the at least two connecting edges comprise three connecting edges, and the three connecting edges comprise first, second and third side edges, wherein the third side edge intersects both the first and second side edges.

Advantageously, the container can be transported and stored in the initial flat or planar configuration, thereby saving both space and costs. The container can then be easily converted to the open configuration when needed. Advantageously, the container does not comprise a membrane. Accordingly, the container is relatively cheap to manufacture and can be disposed using the macerator disposal system installed in most hospital facilities, in embodiments in which the liquid is a biological liquid, such as urine.

Preferably, the volume in which liquid is contained is completely defined by the inner surfaces of the first and second faces and the connecting edges.

Preferably, the planar material comprises a waterproof material. For example, the planar material may be coated with the waterproof material. Preferably, the planar material is laminated, for example with the waterproof material. It will be appreciated that the planar material does not need to be coated on both sides. Preferably, the inner surfaces of the first and second faces comprise the waterproof material. Preferably, the waterproof material comprises a polymer. Preferably, the waterproof material comprises polyethylene.

The planar material may comprise bamboo. The planar material may comprise a rigid board comprising bio-degradable pulp. The planar material may comprise corrugated board. Preferably, the planar material comprises cardboard. Accordingly, in a preferred embodiment, the planar material comprises polyethylene-coated cardboard.

Advantageously, the laminated material is designed to withstand contact with liquid for extended periods of time, and retain its shape without going soggy and losing its shape or form, which would cause leaks. Accordingly, and preferably, the container is capable of holding the liquid for at least three, four or five hours without leakage.

Preferably, the first and second faces comprise one continuous piece of planar material. Accordingly, preferably the at connecting edges comprise a first folded edge and at least one further connecting edge, wherein the first folded edge is defined as a first fold in the planar material which separates the first face from the second face.

Preferably, the first face comprises a first front edge, which is at least partially unconnected to the second face. The first front face may intersect the first and second side edges. However, in a preferred embodiment, the first front edge extends between the first side edge and the second side edge. Preferably, the second face comprises a second front edge, which is at least partially unconnected to the first face. More preferably, the second front edge is unconnected to the first face. The second front face may intersect the first and second side edges. Preferably, when the container is in the open configuration, the front edges at least partially define an opening allowing access to the volume. Preferably, the opening is integral to the remainder of the container.

Preferably, the container is asymmetrical. Preferably, in the planar configuration the opening is positioned substantially adjacent to one of the interconnecting edges and spaced apart from another of the interconnecting edges. Accordingly, in the planar configuration the opening may be positioned substantially adjacent to the first edge and spaced apart from the second edge. Alternatively, in the planar configuration the opening may be positioned substantially adjacent to the second edge and spaced apart from the first edge.

Preferably, due to the asymmetry of the container, in the open configuration the opening may be substantially central.

Advantageously, the locking means enables the container to be held in the open configuration without the application of glue or external fasteners.

Preferably, when the container is in the open configuration a portion of the container comprises a base, and the base is configured to enable the container to rest on a flat surface. Preferably, the container may hold a volume of liquid while the base is resting on a flat surface.

Advantageously, liquid can be put in the container and the container can then be left on a surface prior to be transported or disposed of.

Preferably, the second face is larger than the first face. Accordingly, when the container is in the planar configuration, the first front edge may be adjacent to the inner surface of the second face.

Advantageously, when the container is in the open configuration, a first portion of the first front edge defines a first lip and a first portion of the second front edge defines a second lip. Preferably, the second lip extends beyond the footprint of the first face. Advantageously, the opening is easily accessible to a user.

Preferably, the first face comprises the tab, which is defined by the shape of the first front edge and is preferably unconnected to the second face. Preferably, the tab comprises a neck portion, which is connected to the remainder of the first face, and a head portion which is adjacent to the neck portion and comprises a maximum width which is greater than the width of the neck portion. Preferably, the tab is disposed substantially centrally between the first side edge and the second side edge.

Preferably, the second face comprises the first aperture, which defines a shape which corresponds to the shape of the head of the tab, and allows the head to fit therethrough. Preferably, the first aperture comprises a substantially straight edge which defines a first indent or slot which is wider than the width of the neck of the tab. Preferably, the first indent or slot is narrower than the width of the head of the tab. Preferably, the first aperture is disposed substantially adjacent to the second front edge, and is preferably disposed substantially adjacent to the first side.

Preferably, the second face comprises a cut-out or slot configured to receive a distal end of the tab, and the tab is sized to extend into the cut-out or slot when the container is in the open configuration.

Advantageously, the cut-out or slot holds the tab in place thereby locking the container in the open configuration.

In a most preferred embodiment, the first aperture defines a first indent or slot configured to receive the neck of the tab and the second face comprises a cut-out or slot configured to receive a distal end of the tab.

Advantageously, when the container is in the open configuration the tab is held at two points ensuring that the container is locked in the open position.

Preferably, the first face comprises a connected section, which is adjacent to the first face and substantially adjacent to the aperture. Preferably, the inner surface of the connected section is joined to a first portion of the inner surface of the second face. Preferably, the connected section is adjacent to the first side.

In one embodiment, the inner surface of the connected section is joined to the first portion inner surface of the second face by means of an adhesive.

However, in a preferred embodiment, the inner surface of the connected section is joined to the first portion of the inner surface of the second face by heat sealing.

Preferably, when the container is in the planar configuration, the tab is disposed spaced apart from the first aperture. Preferably, when the container is in the open configuration, the tab is disposed adjacent to the first aperture, such that the head of the tab may be passed through the first aperture and the neck may be held in the first indent, thereby locking the container in the open configuration.

Advantageously, the first aperture can be used as a handle to lift the container when it is in the open configuration. Accordingly, the container can be easily transported when it is holding a liquid in the volume. Furthermore, the carrying handle is part of the locking mechanism which holds the container in the open configuration.

The neck of the tab may comprise a tab crease which extends across the width thereof. Advantageously, this better enables the tab to be bent, thereby enabling the head of the tab to be passed through the first aperture more easily.

Preferably, when the container is in the open configuration, the first and/or second faces define a wall which extends between the first lip and second lip and further defines the opening. Preferably, the wall comprises a portion of the second face adjacent to the first aperture and/or a portion of the first face adjacent to the tab. Preferably, the wall comprises the portion of the second face adjacent to the first aperture and the portion of the first face adjacent to the tab.

The container may comprise a strengthening section configured to strengthen the first aperture.

The second face may be provided with the strengthening section, which comprises a further layer of the planar material, wherein the inner surface of the strengthening section is joined to a second portion of the inner surface of the second face, wherein the second portion is at least adjacent to the first aperture.

Alternatively, the first face may comprise the strengthening section, wherein an inner surface of the strengthening section is configured to be disposed adjacent to an inner surface of the second face at least adjacent to the first aperture when the container is in the open configuration. Preferably, an inner surface of the strengthening section is configured to be disposed adjacent to an inner surface of the second face at least adjacent to the first aperture when the container is in flat configuration.

Advantageously, the strengthening section strengthens the aperture and thereby reduces the risk that the aperture will bend or tear causing the locking means to fail and/or causing a user to drop the container when they are using the aperture as a handle.

Preferably, the strengthening section comprises a second aperture which is sized and shaped substantially the same as the first aperture, such that the second aperture overlies the first aperture.

Advantageously, the edges of both apertures are strengthened due to the combined strength of the second face and the strengthening section.

In one embodiment, the inner surface of the strengthening section is joined to the second portion of the inner surface of the second face by means of an adhesive. However, in a preferred embodiment, the inner surface of the strengthening section is joined to the second portion of the inner surface of the second face by heat sealing.

In a more preferred embodiment, the inner surface of the strengthening section is overlies a portion of second face but is not joined thereto.

Preferably, one continuous piece of planar material comprises the second face and the strengthening section. Preferably, the second front edge comprises a second folded edge, which is defined as a second fold in the planar material, which defines the second face and the strengthening section.

Preferably, when the container is in the open configuration, the second lip comprises a folded edge. In one embodiment this may comprise the second folded edge. In an alternative embodiment, the container may comprise a flap which may be folded manually to define the folded edge. Advantageously, due to the folded edge, the second lip comprises a softened edge.

Preferably, the first face, second face and strengthening section are sized such that when the container is in the planar configuration the footprint of the strengthening section does not overlap with the footprint of the first face. Preferably, the second fold is folded such that when the container is in the planar configuration the strengthening section lies in the same plane as the first face.

Advantageously, the height of the container when in the planar configuration is approximately equal to the width of two pieces of the planar material. Preferably, the height of the container when in the planar configuration is less than 10 mm. More preferably, the height of the container when in the planar configuration is less than 9 mm, 8 mm, 7 mm, 6 mm, 5 mm or 4 mm. Most preferably, the width of the container is less than 3 mm.

Preferably, when in the planar configuration, the container has a maximum length of between 200 mm and 500 mm. More preferably, the maximum length of the container is between 250 mm and 400 mm. Most preferably, the maximum length of the container is between 300 mm and 350 mm.

Preferably, when in the planar configuration, the container has a maximum width of between 100 mm and 300 mm. More preferably, the maximum width of the container is between 150 mm and 250 mm. Most preferably, the maximum width of the container is between 175 mm and 225 mm.

Preferably, the first and second faces each comprise a node, wherein when the container is in the planar configuration, the first face node is substantially adjacent to the second face node, and when the container is in the open configuration, the first face node is spaced apart from the second face node, and the distance between the nodes defines the maximum height of the container when in the open configuration.

The first and second face nodes may be disposed centrally between the first and second side edges. However, in a preferred embodiment, the first and second face nodes are disposed closer to the second side edge than the first side edge.

Accordingly, the ratio of the distance that the first and second face nodes are disposed between the first side edge and the second side edge may be between 120:60 and 91:89. Preferably, the ratio of the distance that the first and second face nodes are disposed between the first side edge and the second side edge is between 110:70 and 92:88. More preferably, the ratio of the distance that the first and second face nodes are disposed between the first side edge and the second edge is between 100:80 and 93:87. In a most preferred embodiment, the ratio of the distance that the first and second face nodes are disposed between the first side edge and the second edge is about 95:85.

Advantageously, the positioning of the node and relative positioning of the tab and aperture, as defined above, cause the positioning of the opening to be in a relatively central position.

Preferably, when in the open configuration, the container has a maximum height which is between 50 mm and 250 mm. More preferably, the maximum height is between 100 mm and 200 mm.

Preferably, the first face of the container comprises a first folding section and the second face comprises a second folding section, wherein when the container is in the planar configuration, the inner surface of the first folding section is adjacent to at least a portion of the inner surface of the second folding section, and when the container is in the open configuration, the first and second folding sections are adjacent to each other, and the inner surfaces of the first and second folding sections define an angle of greater than 45°.

Preferably, the first folding section is adjacent to the first face node. Preferably, the first folding section is substantially adjacent to the tab.

Preferably, the second folding section is adjacent to the second face node. Preferably, the second folding section is adjacent to the first side edge. Preferably, the second folding section is substantially adjacent to the first aperture.

Preferably, when the container is in the open configuration, the first and second folding sections are adjacent to each other, and the inner surfaces of the first and second folding sections define an angle of greater than 90°, or greater than 120°. More preferably, when the container is in the open configuration, the first and second folding sections are adjacent to each other, and the inner surfaces of the first and second folding sections define an angle of greater than 150°. Most preferably, when the container is in the open configuration, the first and second folding sections are adjacent to each other, and the inner surfaces of the first and second folding sections are substantially planar.

Preferably, the first face comprises a third folding section, wherein when the container is in the planar configuration, an inner surface of the third folding section is adjacent to a further portion of the inner surface of the second folding section. Preferably, the third folding section is adjacent to the first folding section, and preferably the third folding section is adjacent to the first side edge.

Preferably, when the container is in the open configuration, the first and third folding sections are adjacent to each other, and outer surfaces of the first and third folding sections define an angle of less than 135°.

Preferably, when the container is in the open configuration, the first and third folding sections are adjacent to each other, and the outer surfaces of the first and third folding sections define an angle of less than 90°, or less than 60°. More preferably, when the container is in the open configuration, the first and third folding sections are adjacent to each other, and the outer surfaces of the first and third folding sections define an angle of less than 30°. Most preferably, when the container is in the open configuration, the outer surfaces of the first and third folding sections are substantially adjacent.

Preferably, the inner surface of the third folding section is joined to the further portion of the inner surface of the second folding section.

In one embodiment, the inner surface of the third folding section is joined to the further portion of the inner surface of the second folding section by means of an adhesive.

However, in a preferred embodiment, the inner surface of the third folding section is joined to the further portion of the inner surface of the second folding section due to heat sealing.

Preferably, when the container is in the planar configuration, the first and second faces comprise a plurality of creases, and the conversion from the planar configuration to the open configuration comprises folding the first and second faces along the plurality of creases, such that when the container is in the open configuration, the first and second faces comprise a plurality of folds.

It will be appreciated that the plurality of folds, when the container is in the open configuration, correspond to the plurality of creases when the container is in the planar configuration.

The first and second faces may comprise at least one flange crease, wherein the or each flange crease extends spaced apart from a peripheral edge of the first and second faces. Preferably, the or each flange crease extends substantially parallel with the peripheral edge. Preferably, the or each flange crease defines a peripheral flange, which comprises the portion which extends between the flange crease and the peripheral edge.

Preferably, the first face comprises at least one first face flange and the second face comprises at least one corresponding second face flange, wherein the inner surface of the or each first face flange is joined to the inner surface of the or each corresponding second face flange thereby defining a flange connecting edge.

In one embodiment the inner surfaces of the corresponding flanges are joined by means of an adhesive.

However, in a preferred embodiment, the inner surfaces of the corresponding flanges are joined due to heat sealing.

It will be appreciated that each of the at least two connecting edges may comprise at least one flange connecting edge.

In embodiments not belonging to the invention where the container comprises one piece of planar material the at least two connecting edges may comprise a first folded edge and at least one flange connecting edge.

Additionally, where the at least two connecting edges comprise three connecting edges then the three connecting edges may comprise a first folded edge and two flange connecting edges.

In a most preferred embodiment, the two flange connecting edges are joined due to heat sealing, and no further sections of the container are heat sealed.

It will be appreciated that the third side edge may comprise the folded edge and first and second side edges may comprise the two flange connecting edges. Alternatively, the first side edge may comprise the folded edge and the second side edge and third side edge may comprise the two flange edges or the second side edge may comprise the folded edge and the first side edge and third side edge may comprise the two flange edges.

The container may comprise at least one connecting vertex, wherein the or each connecting vertex is defined by the point where two connecting edges intersect.

Accordingly, in embodiments which do not belong to the invention where the container comprises two connecting edges the container may comprise a connecting vertex defined by the point where the first and second side edges intersect. Preferably, the first face comprises a first volume crease extending between the connecting vertex and the first face node. Preferably, the second face comprises a second volume crease extending between the connecting vertex and the second face node.

However, in the preferred embodiment, where the container comprises three connecting edges, a first connecting vertex is defined by the point where the first side edge and third side edge intersect and a second connecting vertex is defined by the point where the second side edge and third side edge intersect.

Preferably, the first face comprises first and third volume creases, wherein the first volume crease extends between the first connecting vertex and the first face node and the third volume crease extends between the second connecting vertex and the first face node. Preferably, the first and third volume creases and the third side edge define a first triangular section.

Preferably, the second face comprises second and fourth volume creases, wherein the second volume crease extends between the first connecting vertex and the second face node and the fourth volume crease extends between the second connecting vertex and the second face node. Preferably, the second and fourth volume creases and the third side edge define a second triangular section.

Preferably, the second triangular section corresponds to the size and shape of the first triangular section. Preferably, in the planar configuration an inner surface of the second triangular section is adjacent an inner surface of the first triangular section.

Preferably, the second triangular section defines the base. Advantageously, when the container is in the open configuration it can be left to rest on the second triangular section while holding a liquid in the volume.

Preferably, the second face comprises a fifth volume crease which extends between the first side edge and the second face node.

Preferably, the fifth volume crease and the first side edge define an angle of between 45° and 135°. More preferably, the fifth volume crease and the first side edge define an angle of between 55° and 125°, between 65° and 115°, between 75° and 105° or between 85° and 95°. Most preferably, the fifth volume crease and the first side edge define an angle of about 90°.

Preferably, the second and fifth volume creases and a first portion of the first side edge define a third triangular section, wherein the first portion of the first side edge extends between the second volume crease and the third volume crease. Preferably, the third triangular section is adjacent the second triangular section in embodiments where this is present. Preferably, the third triangular section comprises a scalene triangle. Preferably, the third triangular section comprises a right angled triangle. Most preferably, the third triangular section comprises a right angled scalene triangle.

Preferably, the second face comprises a sixth volume crease which extends between the second front edge and the second face node.

Preferably, the fifth and sixth volume creases define an angle of between 45° and 135°. More preferably, the fifth and sixth volume creases define an angle of between 55° and 125°, between 65° and 115°, between 75° and 105° or between 85° and 95°. Most preferably, the fifth and sixth volume creases define an angle of about 90°.

Preferably, the fourth and sixth volume creases, the second side edge and a first portion of the second front edge, which extends between the sixth volume crease and the second side, define a further second face section. Preferably, the further second face section is adjacent the second triangle section in embodiments where this is present.

Preferably, the second face comprises a seventh volume crease which is disposed between the third and fourth volume creases and extends between the second face node and the first side edge or the second front edge.

Preferably, the seventh volume crease extends between the second face node and a first front vertex, which is defined by the intersection of the first side edge and the second front edge.

Preferably, the sixth and seventh volume creases and a second portion of the second front edge, which extends between the sixth volume crease and the first side edge, define a handle section. Preferably, the handle section comprises the first aperture. Preferably, the handle section is adjacent the further second face section.

Preferably, the fifth and seventh volume creases define an angle of between 10° and 90°. More preferably, the fifth and seventh volume creases define an angle of between 15° and 80°, between 20° and 70°, between 25° and 70°, between 30° and 60°, between 40° and 50°. Most preferably, the fifth and seventh volume creases define an angle of about 45°.

Preferably, the fifth and seventh volume creases and a second portion of the first side edge, which extends between the fifth volume crease and the second front edge, define the second folding section. Preferably, the second folding section is adjacent the handle section. Preferably, the second folding section is adjacent the third triangular section. Preferably, the second folding section comprises a right angled triangle. Most preferably, the second folding section comprises a right angled isosceles triangle.

Preferably, the first face comprises an eighth volume crease, which extends between the first side edge and the first face node.

Preferably, the eighth volume crease and the first side edge define an angle of between 45° and 135°. More preferably, the eighth volume crease and the first side edge define an angle of between 55° and 125°, between 65° and 115°, between 75° and 105° or between 85° and 95°. Most preferably, the eighth volume crease and the first side edge define an angle of about 90°.

Preferably, the first and eighth volume creases and the first portion of the first side edge define a fourth triangular section. Preferably, the first portion of the first side edge extends between the first volume crease and the eighth volume crease. Preferably, the fourth triangular section is adjacent the first triangular section in embodiments where this is present. Preferably, the fourth triangular section comprises a scalene triangle. Preferably, the fourth triangular section comprises a right angled triangle. Most preferably, the fourth triangular section comprises a right angled scalene triangle. Preferably, the fourth triangular section corresponds to the size and shape of the third triangular section. Preferably, when the container is in the planar configuration the inner surface of the fourth triangular section is adjacent the inner surface of the third triangular section.

Preferably, the first face comprises a ninth volume crease which extends between the first front edge and the first face node.

Preferably, the first and ninth volume creases define an angle of between 90° and 270°. More preferably, the first and ninth volume creases define an angle of between 110° and 250°, between 130° and 230°, between 150° and 210° or between 170° and 190°. Most preferably, the first and ninth volume creases define an angle of about 180°.

Preferably, the third and ninth volume creases, the second side edge and a first portion of the first front edge, which extends between the ninth volume crease and the second side, define a further first face section. Preferably, the further first face section is adjacent the first triangular section in embodiments where this is present.

Preferably, the first face comprises a further node. Preferably, the further node is substantially adjacent the first front edge. Preferably, the further node is adjacent the first folding section. Preferably, the further node is adjacent the third folding section, in embodiments where this is present. Preferably, the first face comprises a tenth volume crease which extends between the first face node and the further node. Preferably, the first face comprises an eleventh volume crease which extends between the first front edge and the further node.

Preferably, the tenth and eleventh volume creases define an angle of between 45° and 135°. More preferably, the tenth and eleventh volume creases define an angle of between 55° and 125°, between 65° and 115°, between 75° and 105° or between 85° and 95°. Most preferably, the tenth and eleventh volume creases define an angle of about 90°.

Preferably, the eleventh volume crease and the first front edge define an angle of between 45° and 135°. More preferably, the eleventh volume crease and the first front edge define an angle of between 55° and 125°, between 65° and 115°, between 75° and 105° or between 85° and 95°. Most preferably, the eleventh volume crease and the first front edge define an angle of about 90°.

Preferably, the ninth volume crease, a second portion of the first front edge, which extends from the ninth volume crease to the tab crease, the tab crease, a third portion of the first front edge, which extends from the tab crease to the eleventh volume crease, the eleventh volume crease and the tenth volume crease define a tab connecting section.

Preferably, the first front face comprises a twelfth volume crease which extends between the first front edge and the further node.

Preferably, the twelfth volume crease and the first front edge define an angle of between 10° and 90°. More preferably, the twelfth volume crease and the first front edge define an angle of between 15° and 80°, between 20° and 70°, between 25° and 70°, between 30° and 60°, between 40° and 50°. Most preferably, the twelfth volume crease and the first front edge define an angle of about 45°.

Preferably, the eleventh and twelfth volume creases and a fourth portion of the first front edge, which extends between the eleventh and twelfth volume creases, define a further connecting section. Preferably, the further connecting section is adjacent the tab connecting section. Preferably, the further connecting section is adjacent the connected section. Preferably, the further connecting section comprises a right-angled triangle. Preferably, the further connecting section comprises an isosceles triangle. Most preferably, the further connecting section comprises a right angled isosceles triangle.

Preferably, the first face comprises a second indent, wherein the second indent extends between the first front vertex to the further node. Preferably, the twelfth volume crease, a fifth portion of the first front edge, which extends between the twelfth volume crease and the second indent, and the second indent define the connected section.

Preferably, the second indent and the first side edge define an angle of between 10° and 90°. More preferably, the second indent and the first side edge define an angle of between 15° and 80°, between 20° and 70°, between 25° and 70°, between 30° and 60°, between 40° and 50°. Most preferably, the second indent and the first side edge define an angle of about 45°.

Preferably, the second indent is adjacent at least a portion of the seventh crease.

Advantageously, the second indent allows the container to be converted between the first and second configurations without causing any excess strain within the first face where the inner surface is joined to the inner surface of second face.

The container may comprise a thirteenth volume crease which extends between the further node and the first side.

However, in a preferred embodiment the first face comprises a third indent, which extends from the further node towards the first side edge, and a thirteenth volume crease, which extends from the third indent towards the first side edge, and the third indent and the thirteenth volume crease define an angle of approximately 180° with.

Preferably, the second and third indents define an angle of between 45° and 135°. More preferably, the second and third indents define an angle of between 55° and 125°, between 65° and 115°, between 75° and 105° or between 85° and 95°. Most preferably, the second and third indents define an angle of about 90°.

Preferably, the thirteenth volume crease and the first side edge define an angle of between 10° and 90°. More preferably, the thirteenth volume crease and the first side edge define an angle of between 15° and 80°, between 20° and 70°, between 25° and 70°, between 30° and 60°, between 40° and 50°. Most preferably, the thirteenth volume crease and the first side edge define an angle of about 45°.

Preferably, the eighth, tenth and thirteenth volume creases and the third indent define the first folding section. Preferably, the first folding section is adjacent the tab connecting section. Preferably, the first folding section is adjacent the fourth triangular section. Preferably, the first folding section comprises an isosceles triangle. Preferably, the first folding section comprises a right angled triangle. Preferably, the first folding section comprises a right angled isosceles triangle. Preferably, the first folding section is a mathematically similar shape to the second folding section. Preferably, the inner surface of the first folding section comprises approximately half the area of the inner surface of the second folding section.

Preferably, thirteenth volume crease, the second and third indents and the second portion of the first side define the third folding section. Preferably, the third folding section is adjacent the first folding section. Preferably, the third folding section is substantially adjacent the connected section. Preferably, the third folding section comprises an isosceles triangle. Preferably, the third folding section comprises a right angled triangle. Preferably, the third folding section comprises a right angled isosceles triangle. Preferably, the third folding section is a mathematically similar shape to the second folding section. Preferably, the inner surface of the third folding section comprises approximately half the area of the inner surface of the second folding section. Preferably, the third folding section corresponds in size and shape to the first folding section.

Preferably, the substantially opposing forces comprise a first force and a second force, which substantially opposes the first force.

Preferably, the container is configured to be converted from the planar configuration to the open configuration upon application of the opposing forces, where the first force is applied to the first side edge. More preferably, the container is configured to be converted from the planar configuration to the open configuration upon application of the opposing forces, where the first force to be applied to the first side, substantially adjacent the first front edge. Most preferably, the container is configured to be converted from the planar configuration to the open configuration upon application of the opposing forces, when the first force to be applied to the first side, adjacent the third folding section.

Preferably, the container is configured to be converted from the planar configuration to the open configuration upon application of the opposing force, when the second force is applied to the second side edge. Preferably, the container is configured to be converted from the planar configuration to the open configuration upon application of the opposing force, when the second force is applied to the second side edge, substantially adjacent the first front edge.

Preferably, the container comprises at least two arrows indicating the direction in which the opposing forces should be applied to convert the container from the planar configuration to the open configuration.

The arrows could be printed on an outer surface of the container.

However, in a preferred embodiment the arrows are defined by apertures in the first face.

Preferably, the first face comprises a first arrow adjacent the first side and substantially adjacent the first front edge. Preferably, the third folding section comprises the first arrow.

Preferably, the first face comprises a second arrow adjacent the second side and substantially adjacent the first front edge. Preferably, the further first face section comprises the second arrow.

Advantageously, the arrows will direct a user wanting to convert the device from a planar configuration to the open configuration. As the arrows are provided as apertures, and not printed, this reduces costs in the manufacturing process.

Preferably, the container is configured to hold at least 100 ml of liquid. More preferably, the container is configured to hold at least 200 ml, 300 ml, 400 ml, 500 ml, 600 ml, 700 ml, 800 ml or 900 ml of liquid. Most preferably, the container is configured to hold at least 1000 ml of liquid.

The container may comprise a transparent window and a corresponding volume scale. The first triangular section may comprise the transparent window and corresponding scale. Alternatively, the second triangular section may comprise the transparent window and corresponding scale.

Advantageously, the transparent window and corresponding scale allows a user to measure the amount of liquid in the container.

The container may comprise a disposable container for use in holding liquid for drinking; for gardening purposes; for engineering applications (e.g. lubricants or cleaning liquids); for catering and kitchen applications; or for healthcare applications.

However, in a preferred embodiment, the liquid is urine. Preferably, the container comprises a disposable male urine container. Hence, the container may be used in hospitals or care homes, and the like.

The container of the invention may be used to contain and/or carry liquid.

Thus, also disclosed is the use of the container of the first aspect for containing, storing or carrying liquid.

Also disclosed is a method for containing, storing or carrying liquid, the method comprising inserting liquid in the container of the first aspect.

Furthermore there is disclosed a method of manufacturing a liquid container, the method comprising:
- cutting a planar material to produce a cut-out, and pre-creasing the planar material with a plurality of creases, wherein the shape of the cut-out and the plurality of creases define a first face, a second face, at least a pair of corresponding peripheral flanges and a locking mechanism,;folding the material about a central crease thereby causing an inside surface of the first face to be substantially adjacent to an inside surface of the second face, and causing inside surfaces of the corresponding flanges to be substantially adjacent to each other; and
   sealing the inside surfaces of the corresponding flanges together, wherein the locking means comprises a tab disposed in the first face 8, and a first aperture disposed in the second face, wherein the first aperture is configured in use to receive the tab, thereby holding the container in the open configuration, and characterised in that the container comprises three connecting edges, and the three connecting edges comprise first, second and third side edges, wherein the third side edge intersects both the first and second side edges.

Advantageously, the container produced may be flat without the need to carry out any further steps to flatten the container prior to storage and transportation.

Preferably, the liquid container made using the method comprises the container of the first aspect.

Preferably, the planar material comprises a rigid planar material. Preferably, the rigid planar material is as described in reference to the first aspect.

Preferably, the shape of the cut-out and the plurality of creases define two pairs of corresponding peripheral flanges. The method comprises sealing the inside surfaces of each of the two pairs of corresponding flanges together. Preferably, the or each pair of heat sealed flanges define a side of the container.

The method comprises heat sealing each of the two pairs of corresponding flanges together, and no further heat sealing is performed. Alternatively, the method may comprise sealing an inside surface of a selected portion of the first face to an inside surface of a selected portion of the second face.

The selected portion of the first face may be at least partially defined by the shape of the cut-out and the plurality of creases. Preferably, the selected portion of the first face is fully defined by the shape of the cut-out and the plurality of creases.

The selected portion of the first face may comprise the connected section and/or the third folding section. Preferably, the selected portion of the first face comprises the connected section and the third folding section

The selected portion of the second face may be at least partially defined by the shape of the cut-out and the plurality of creases.

The selected portion of the second face may comprise a portion of the second folding section and/or a portion of the handle section. Preferably, the selected portion of the second face comprises a portion of the second folding section and a portion of the handle section.

Preferably, the shape of the cut-out and the plurality of creases further define a strengthening section. Preferably, the method comprises folding the material about a strengthening crease thereby causing the inside surface of the strengthening section to be substantially adjacent to an inside surface of a second portion of the second face, and sealing the inside surfaces of the strengthening crease and the second portion of the second face together.

The sealing step may comprise using adhesive.

However, in a preferred embodiment, the sealing step comprises a heat sealing step.

Advantageously, the heat sealing may be carried out in one plane. This ensures that the method of manufacture is simple.

All features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figures, in which:-
**Figure 1** is a plan view from above of one embodiment of a disposable liquid container of the invention shown in a folded, planar configuration;
**Figure 2** is a perspective view of the liquid container of Figure 1 in a partially open configuration;
**Figure 3** is a further perspective view of the liquid container of Figure 1 in the partially open configuration;
**Figure 4** is a perspective view of the liquid container of Figure 1 in a fully open configuration;
**Figure 5** is a perspective view of a left side elevation of the liquid container in the fully open configuration;
**Figure 6** is a perspective view of a right side elevation of the liquid container in the fully open configuration;
**Figure 7** is a perspective view of a front elevation of the liquid container in the fully open configuration;
**Figure 8** is a plan view from above of a cut-out which can be converted into the liquid container of Figures 1 to 7;
**Figure 9** is a plan view from above of a first face portion, and adjacent flanges, of the cut-out of Figure 8;
**Figure 10** is a plan view from above of a second face portion, and adjacent flanges, of the cut-out of Figure 8;
**Figure 11** is a plan view from above of a flap portion of the cut-out of Figure 8;
**Figure 12** is a plan view from above of an alternative embodiment of a disposable liquid container of the invention shown in a folded, planar configuration;
**Figure 13** is a perspective view of the liquid container of Figure 12 in a partially open configuration; and
**Figure 14** is a perspective view of a left side elevation of the liquid container of Figures 12 and 13 in the fully open configuration

### Example 1: Manufacture of a disposable flat-pack liquid container

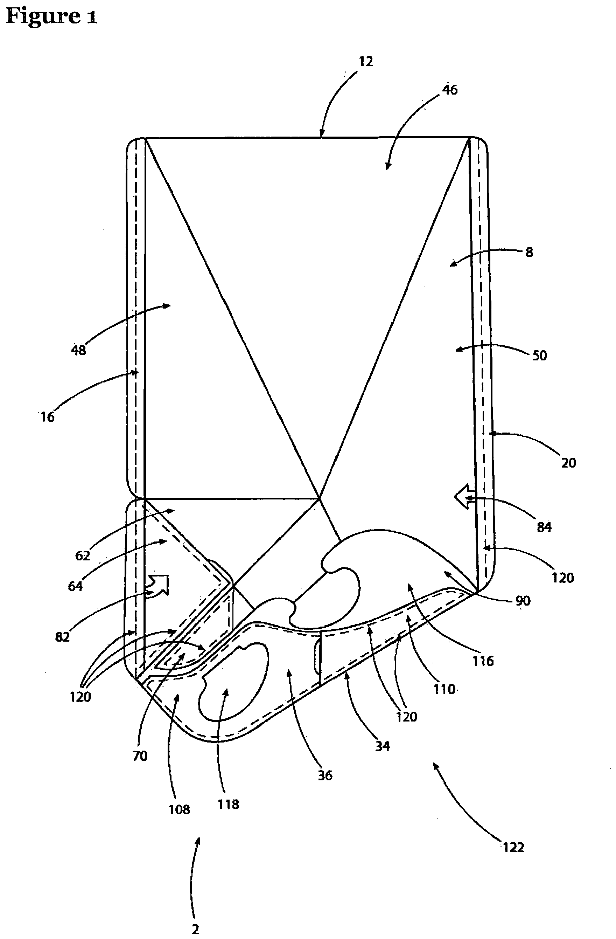

A disposable flat-pack liquid container or bottle 2 is shown in Figures 1 to 7. The bottle 2 can have many different applications, such as those described in Example 5, including storing liquid for drinking, gardening purposes, catering and kitchen applications etc. However, the primary use of the bottle 2 is as a male urine storage container.

One of the advantages conferred by the bottle 2 is that it is stored in a flat, planar configuration 122, as shown in Figure 1. In the flat configuration the bottle has a maximum length of about 330 mm and a width of about 200mm, and the total height is less than 3 mm. The dimensions of the width and the length have been constrained by the sealing plate on the heat sealer used by the inventors. However, it will be appreciated that alternative heat sealers could be used which would allow the length and width to be varied.

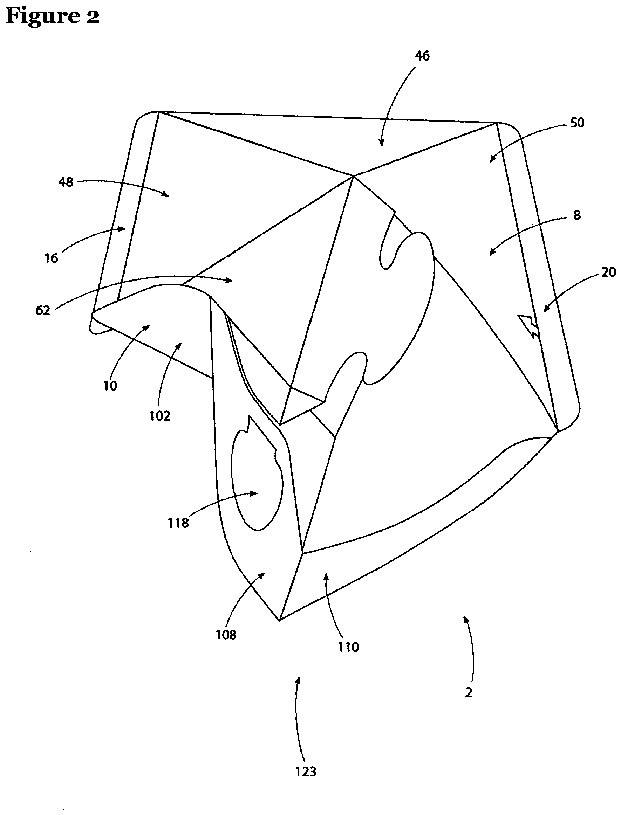

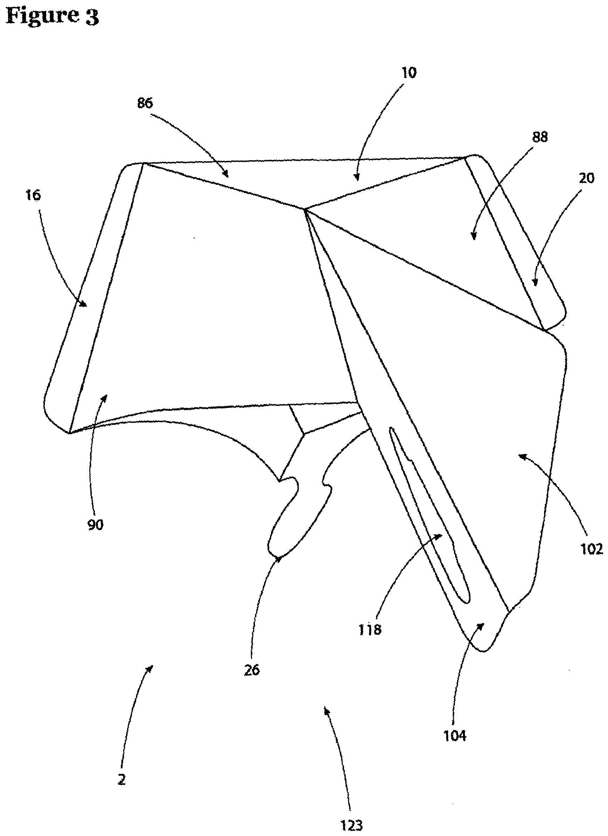

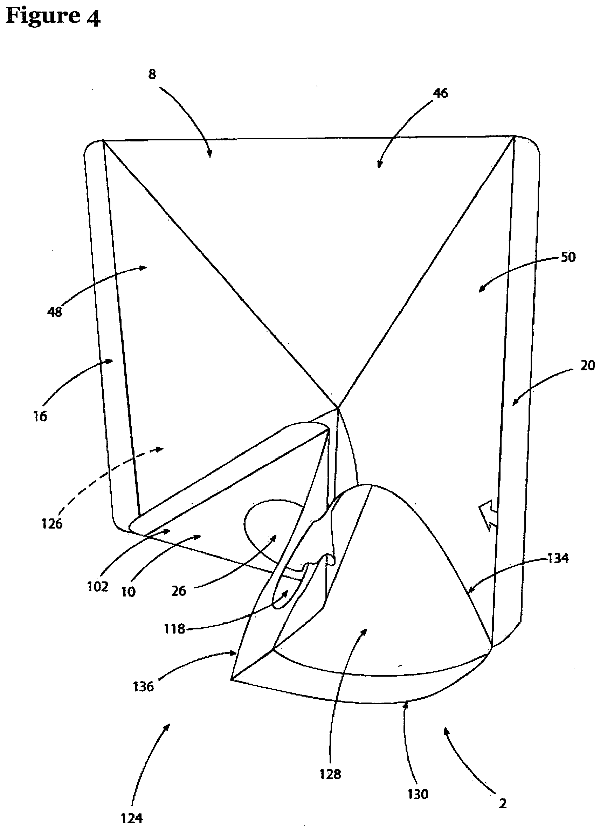

The bottle 2 can then be readily converted to an open configuration 124, as shown in Figures 4 to 7, in which it holds liquid, such as water (or urine). In this configuration it will have a maximum height of about 135 mm, which is defined by the length of a tenth volume crease 106, which is described below. It will be appreciated that as the bottle 2 is converted from the flat configuration 122 into the open configuration 124, it must pass through a partially open configuration 123, which is shown in Figures 2 and 3.

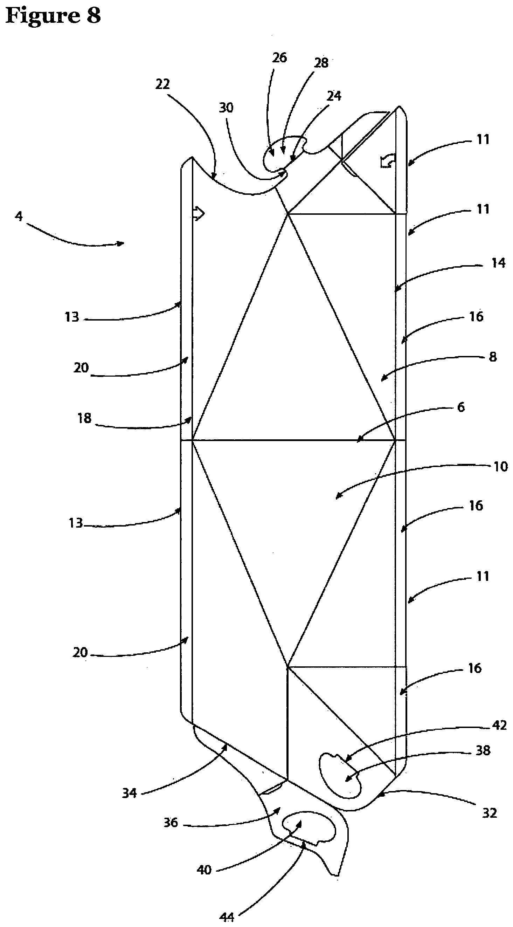

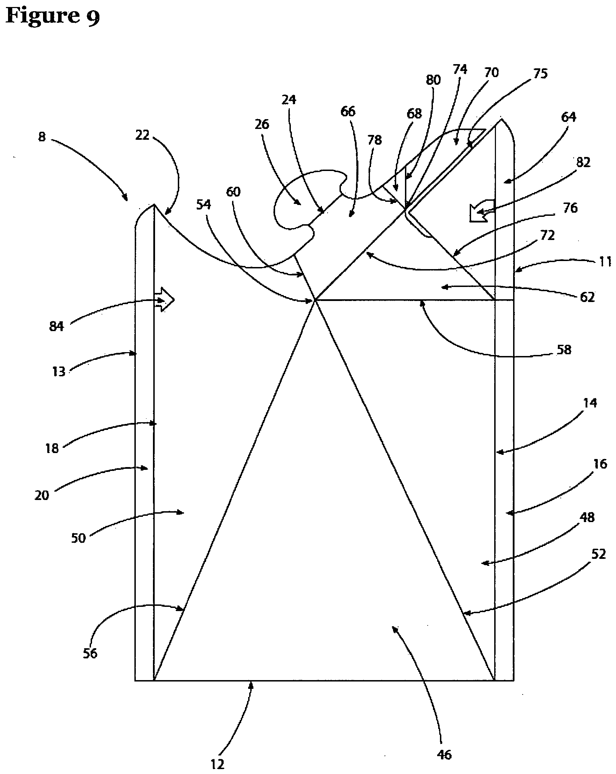

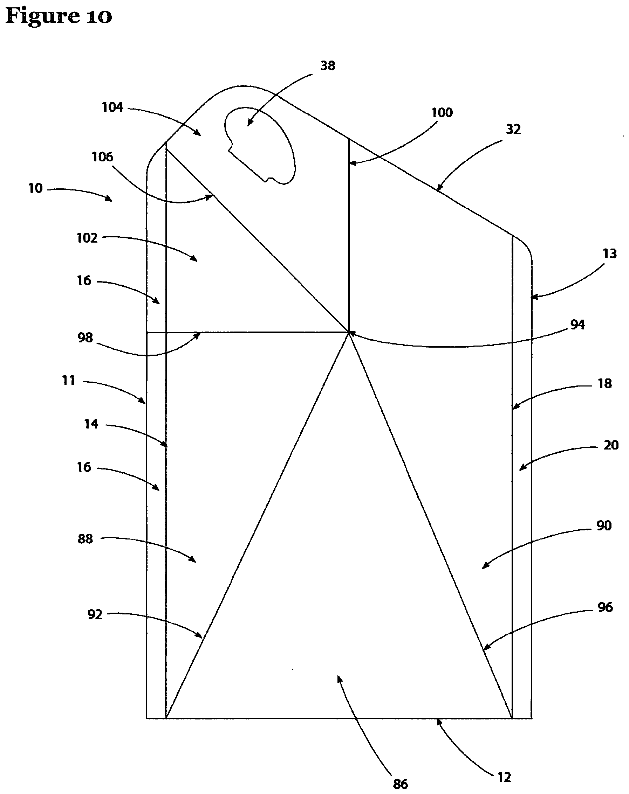

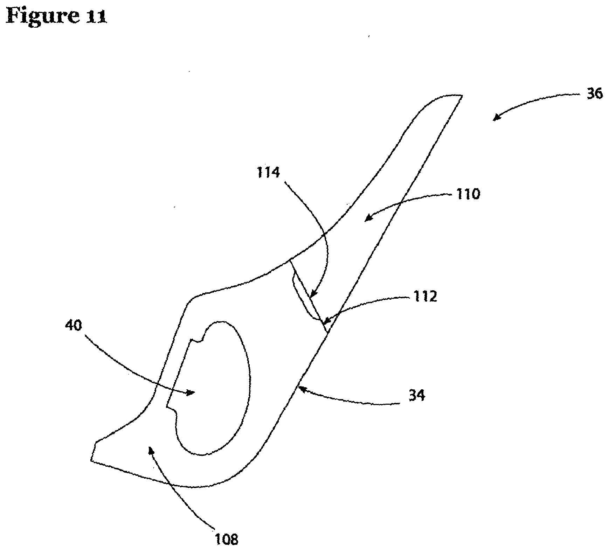

Referring to Figure 8, the bottle 2 is made from a single piece of polyethylene (PE) coated cardboard which is die-cut to form a planar cut-out 4. The laminated material is designed to withstand contact with liquid for extended periods of time, and retain its shape without going soggy and losing its shape or form, which would cause leaks. The cut-out 4 is pre-creased, having a central crease 6, which extends along the centre thereof dividing the cut-out 4 into a first face 8 (shown on the right hand side of Figure 8) and a second face 10 (shown on the left hand side of Figure 8). Extending diagonally across one end of the second face 10, the cut-out 4 also includes a flap crease 34, along which the cut-out 4 is folded to create a distal flap 36. Hence, while it will be appreciated that the cut-out 4 consists of one piece of card in the embodiment illustrated, the central crease 6 and flap crease 34 define three connected portions in the cut-out 4, namely the first face 8 (as shown in Figure 9), the second face 10 (as shown in Figure 10), and the flap 36 (as shown in Figure 11). To better illustrate the various features of each of these portions they are illustrated separately in Figures 9 to 11. However, in practice, these portions are connected.

Turning now to the features defined by the shape of the cut-out 4 and the location of the creases. Referring again to Figure 8, the central crease 6 separates the first face 8 from the second face 10. Extending spaced about 10 mm apart from, and parallel with the left and right peripheral edges 11, 13 of the first and second faces 8, 10, there are disposed left and right edge creases 14, 18, which create left and right flanges 16, 20, respectively. The cut-out 4 is folded over central crease 6 to produce the bottle 2 in its flat configuration 122, as shown in Figure 1, such that the central crease 6 then defines a third side edge 12. Left and right flanges 16, 20 on the first and second faces 8, 10 are stuck together, due to a heat sealing step, such that the planar configuration of the bottle 2 is sealed along three edges, i.e. the third side edge 12, and the left and right flanges 16, 20.

The first face 8 is provided with a curved front edge 22. One section of the edge 22 is inwardly curved (i.e. concave), which leads into another curved section (i.e. convex) which forms a tab 26. The tab 26 comprises a rounded head 28 and a pinched neck section 30, along which extends a tab crease 24, which separates the tab head 28 from the rest of the first face 8 of the cut-out 4.

The second face 10 is also provided with a curved front edge 32, which is outwardly curved (i.e. convex). The front edge 32 is partially defined by the shape of the cut-out 4 and partially defined by the flap crease 34 which separates the second face 10 from the flap 36. A first rounded aperture 38 is disposed in a section of the second face 10 substantially adjacent to the front edge 32 and the left edge crease 14. To reduce the risk of tearing, the first aperture 38 is disposed a minimum of 10 mm from the first front edge 32. A second aperture 40 is disposed in the flap 36. The first and second apertures 38, 40, when aligned with each other, together define a shape which roughly corresponds to the shape of the head 28 of the tab 26, but is slightly larger to allow the head 28 to fit therethrough. Additionally, each aperture 38, 40 includes a straight edge 42 along one side thereof which defines an indent 44 which is slightly wider than the width of the neck 30 of the tab 26. The first and second apertures 38, 40 are shaped and disposed on the cut-out 4 such that they are mirror images of each other and the flap crease 34 defines the line of symmetry.

As can be seen in Figure 8, the right edge crease 18 extends between the curved front edge 22 of the first face 8 and the curved front edge 32 of the second face 10. Similarly, the left edge crease 14 extends between an indentation 75, which is adjacent the curved front edge 22 of the first face 8, and the curved front edge 32 of the second face 10. The indentation 75 is defined in more detail below. As will be clear from Figure 8, the first face 8, second face 10 and flap 36 are provided with further creases which divide these features into various sub-sections. To improve clarity, each of these elements is shown separately in Figures 9 to 11, although it will be appreciated in practice that the elements are not detachable from the cut-out 4.

Referring to Figure 9, the first face 8 has three major sections, namely a top central section 46, a top left section 48 and a top right section 50. The top central section 46 is in the shape of a scalene triangle, the edges of which are defined by the third side edge 12, a first volume crease 52, which extends diagonally between the point where the left edge crease 14 and the third side edge 12 meet and a first face node 54, and a second volume crease 56, which extends diagonally between the point where the right edge crease 18 and the third side edge 12 meet and the first face node 54. For reasons that will be explained below, the first face node 54 is disposed closer to the right edge crease 18 than the left edge crease 14. For instance, in the embodiment of the bottle 2 shown in the Figures, the first face node 54 is disposed 85mm from the right edge crease 18 and 95 mm from the left edge crease 14.

The top left section 48 is in the shape of a right angle triangle, the edges of which are defined by the first volume crease 52, the left edge crease 14 and the third volume crease 58, which extends between the left peripheral edge 11 and the first face node 54, and makes a right angle with the left peripheral edge 11. The top right section 50 is an irregular four sided shape, the edges of which are defined by the second volume crease 56, the right edge crease 18, the concave portion of the curved front edge 22, and a fourth volume crease 60 which extends between the curved front edge 22 and the first face node 54.

The first face 8 also comprises six minor sections. One of the minor sections is the tab 26, which has already been described above, and the remaining five minor sections are a first isosceles triangle section 62, a second isosceles triangle section 64, a first fixing portion 66, a second fixing portion 68 and a third fixing portion 70.

The first isosceles triangle section 62, is in the shape of a right angled isosceles triangle, and is disposed adjacent to the top left section 48 and its longest edge is defined by the third volume crease 58. The remaining two edges are of equal length, one of which is defined by a fifth volume crease 72 which extends between the first face node 54 and a second top node 74. The remaining edge is defined in part by a portion of the indentation 75, which is defined by an L-shape in the cut-out 4, and in part by a triangle crease 76 which extends from the point where the left edge crease 14 and third volume crease 58 meet and towards the second top node 74. The second isosceles triangle section 64 is disposed adjacent to the first isosceles triangle section 62, and is roughly identical to it in size and shape, i.e. it also comprises a right-angled isosceles triangle. The longest edge of the second isosceles triangle section 64 is defined by the left edge crease 14. The remaining two edges are of equal length and defined by the indentation 75 and the triangle crease 76.

It will be appreciated that each of the first and second isosceles triangle sections 62, 64 can be viewed as defining a quartile of a square, and the relative positions of the first and second isosceles triangle sections 62, 64 means that together they define one half of the square.

The first fixing portion 66 is an irregular four sided shape and is disposed between the top right section 50, the first isosceles triangle section 62 and the tab 26. The sides of the first fixing portion 66 are defined by the fourth volume crease 60, the fifth volume crease 72, the curved front edge 22 in combination with the tab crease 24, and a first fixing crease 78, which extends from the curved front edge 22 to the second top node 74. The first fixing crease defines an angle of about 90° with the curved front edge 22.

The second fixing portion 68 is roughly in the shape of a right angled isosceles triangle and is disposed adjacent to the first fixing portion 66. The sides of roughly equal length of the second fixing portion are defined by the first fixing crease 78 and the curved front edge 22. The longest side is defined by a second fixing crease 80, which extends from the curved front edge 22 to the second top node 74.

The third fixing portion 70 is an irregular elongate shape, which is disposed adjacent to and extends away from second fixing portion 68. The third fixing portion 70 is separated from the second isosceles triangle section 64 due to the indentation 75 which defines a gap therebetween. The shape of the third fixing portion 70 is defined by the second fixing crease 80, the curved front edge 22, and the indentation 75. It will be appreciated that the indentation 75 provides ample space to allow the second isosceles triangle section 64 to fold towards the first isosceles triangle section 62, and away from the third fixing portion without problem.

An aperture cut out in the shape of a first arrow 82 is provided adjacent the left edge crease 14, in the second isosceles triangle section 64. Similarly, an aperture cut out in the shape of a second arrow 84 is provided adjacent to the right edge crease 18 in the top right section 50. It will be appreciated that the first and second arrows 82, 84 need not be disposed adjacent the left and right edge creases 14, 18, respectively. Accordingly, in an alternative embodiment, the first and second arrows 82, 84 are disposed spaced apart from the left and right edge creases 14, 18, respectively, by about 5 mm (not illustrated).

Referring now to Figure 10, the second face 10 comprises of three major sections, namely a bottom central section 86, a bottom left section 88 and a bottom right section 90. The bottom central section 86 corresponds to the top central section 46 in the first face 8. Hence, it is in the shape of a scalene triangle, the edges of which are defined by the third side edge 12, a sixth volume crease 92, which extends between the point where the left edge crease 14 and the third side edge 12 meet and a second face node 94, and a seventh volume crease 96, which extends between the point where the right edge crease 18 and the third side edge 12 meet and the second face node 94. As with the first face node 54, the second face node 94 is disposed closer to the right edge crease 18 than the left edge crease 14.

The bottom left section 88 corresponds to the top left section 48. Accordingly, it is in the shape of a right angled triangle, the edges of which are defined by the sixth volume crease 92, the left edge crease 14 and an eighth volume crease 98, which extends at a right angle from the left edge crease 14 to the second face node 94. The bottom right section 90 differs slightly in shape to the top right section 50. It is an irregular four sided shape, the edges of which are defined by the seventh volume crease 96, the right edge crease 18, the curved front edge 32 and a ninth volume crease 100 which extends from the curved front edge 32 to the first second face node 94, and defines a right angle with the eighth volume crease 98.

The second face 10 also comprises two minor sections, namely a third isosceles triangle section 102 and a first handle section 104. The third isosceles triangle section 102 is disposed adjacent to the bottom left section 88, it is right-angled isosceles triangle, and roughly corresponds to the combined shape and size of the first isosceles triangle section 62 and the second isosceles triangle section 64. The sides of equal length of the third isosceles triangle section 102 are defined by the eighth volume crease 98 and the left edge crease 14. The longest side is defined by the tenth volume crease 106, which extends from where the left edge crease 14 meets the curved front edge 32 to the second face node 94. As mentioned above, the tenth volume crease is about 35 mm long. The first handle portion 104 contains the first aperture 38, and is disposed between the third isosceles triangle section 102 and the bottom right section 90. The sides of the handle section are defined by the ninth volume crease 100, the tenth volume crease 106 and the curved front edge 32.

Referring to Figure 11, the flap 36 consists of two portions, namely a second handle section 108, by which the bottle 2 can be carried when filled with liquid, and a further tapered section 110 extending therefrom. The second handle section 108 is defined by the shape of the cut-out, the flap crease 34, a further crease 112 which divides the tapered section 110 from the handle section 108. A further small elongated aperture 114 extends along the further crease 112, and provides ample space to allow the flap 36 to fold towards the tapered section 110 without problem. As can be seen in Figure 8, the further crease 112 extends from the flap crease 34 from the point where the ninth volume crease 100 intersects the flap crease 34. The direction in which the further crease 112 extends is the mirror of the direction of the ninth volume crease 100 where the flap crease 34 is the line of symmetry. The further section 110 is adjacent to the second handle section 108 and its shape is defined by the shape of the cut-out, the flap crease 34, the further crease 112 and the further aperture 114.

In order to form the bottle 2, the cut-out 4 is folded along the central crease 6 so that the first face 8 overlies the second face 10, and the polyethylene coated surfaces 116 of the cut-out 4 face each other. It will be understood that the top central section 46 will directly overlie the bottom central section 86, and the top left section 48 will directly overly the bottom left section 88. The first isosceles triangle section 62 and the second isosceles triangle section 64 will also directly overlie the third isosceles triangle section 102. Additionally, the top right section 50 will overlie the bottom right section 90, but since the bottom right section 90 is larger than the top right section 50, it will extend beyond the top right section 50 , as shown in Figure 1.

The cut-out 4 is also folded along the flap crease 34 so that the flap 36 overlies the second face 10. It will be appreciated that the second handle section 108 will partially overlie the first handle section 104, and the second aperture 40 will directly overly the first aperture 38 to create handle 118. It will be appreciated that the handle 118 is reinforced due to the two layers which have been used to create it. The further section 110 will partially overlie the bottom right section 90.

The folded cut-out 4 is then heat-sealed along the left flanges 16, the right flanges 20, and around the flap 36, the second isosceles triangle section 64 and the third fixing portion 70 to produce the planar bottle 2. The heat-seal points 120 are shown as dashed lines in Figure 1. Accordingly, the bottle 2 is flat when it comes off the production line, being the thickness of just two layers of polyethylene board. In the embodiment illustrated, the bottle has a flat area similar to an A4 envelope.

### Example 2: Converting the bottle from the flat configuration to the open configuration

When the bottle 2 has been manufactured, according to example 1, it will be provided in a flat configuration 122, as shown in Figure 1. This flat configuration 122 allows the bottle 2 to be stored and transported easily. However, it needs to be converted to an open configuration 124, as shown in Figures 4 to 7, before it can be used to receive a liquid.

When a person wants to convert the bottle 2 to the open configuration 124 they need to apply pressure at a point which is adjacent to the first arrow 82, and at a second point which is adjacent to the second arrow 84. Accordingly, the arrows 82, 84 indicate where and how the pressure should be applied. The pressure causes the first face node 54 to be displaced away from the second face node 94, defining a volume 126 therebetween. The bottle 2 is shown in a partially open configuration 123 in Figures 2 and 3. As pressure continues to be applied, the first face node 54 will be forced further apart from the second face node 94 until the second isosceles triangle 64 completely overlies the first isosceles triangle 62, preventing the bottle 2 from being opened any further. At this point the bottle 2 is in the open configuration 124.

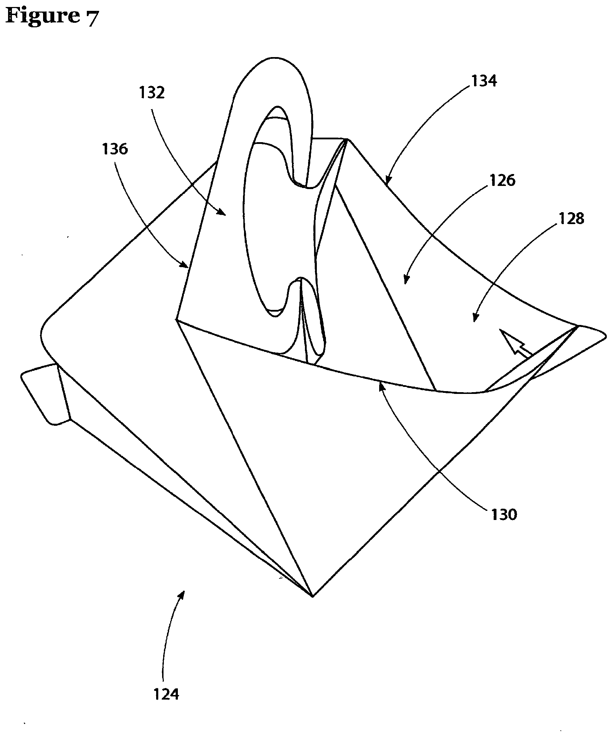

As can be seen in Figure 7, when the bottle 2 is in the open configuration 124 the second face 10 defines a V-shape, when viewed from the front. Additionally, the volume 126 can be accessed via an opening 128. The opening 128 is defined by a bottom lip 130, a side wall 132 and a top lip 134.

It will be appreciated that the bottom lip 130 is defined by the portion of the flap crease 34 which separates the bottom right section 90 from the further section 110. Similarly, the side wall 132 comprises the first handle portion 104 of the second face 10 overlaid with the second handle portion 108 of the flap 36. Accordingly, a front edge 136 of the side wall 132 is defined by the flap crease 34 which separates the first handle portion 104 from the second handle portion 108. The fact that both the bottom lip 130 and front edge 136 are defined by a folded crease is advantageous as it means the edges that will come into contact with a user's penis will have been softened by the fold. It will be appreciated that the top lip 134 is defined by the portion of the front edge 22 which defines an edge of the top right section 50.

Once the bottle 2 is in the open configuration 124, the tab 26 can be folded through the aperture 38 in the handle 118. As can be seen in Figure 7, the handle 118 has been positioned to sit as high above the bottom lip 130 as possible. Due to the shape of the handle 118, the head 28 of the tab 26 is able to pass therethrough, and the neck 30 of the tab 26 can be placed in the indent 44. Accordingly, this creates a lock-and-key type arrangement effectively holding the tab 26 in place and locking the bottle 2 into the open configuration 124.

The bottle 2 made by the inventors and shown in the Figures has a volume 126 which can hold up to 1 litre of liquid when it is in the open configuration. However, it will be appreciated that the capacity of the volume 126 may be varied by varying the dimensions of the cut-out 4.

In use the V-shape of the second face 10 allows the bottle 2 to rest in the gap between a user's legs with the left flange 16 and right flange 20 resting on, or slightly above, the user's legs. As mentioned above, the first face node 54 and the second face node 94 are both disposed closer to the right edge crease 18 than the left edge crease 14. This positioning causes the opening 128 to be provided in a relatively central position. This will mean it is more comfortable for a user to position their penis in the opening 128.

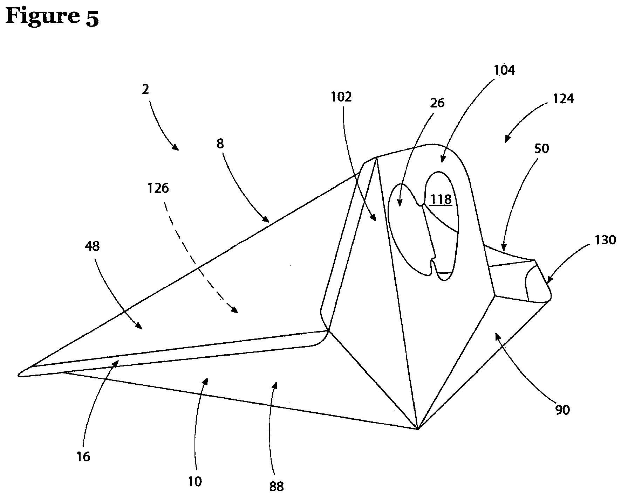

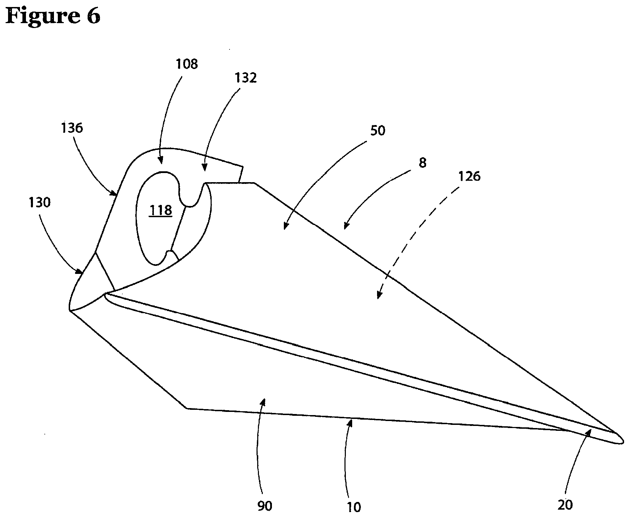

As can be seen in Figures 5 and 6, when the bottle 2 is in the open configuration 124 the bottom central section 86 defines a flat surface on which the bottle 2 can be placed. Accordingly, the bottle 2 can be left on a flat surface after it has been used and prior to it being disposed. After use, the bottle 2 can also be transported by a user by inserting a finger into the handle 118, or simply gripping the handle section 108, and carried with ease.

While not illustrated in the Figures, in another embodiment, the top central section 46 or the bottom central section 86 comprises a transparent window with a scale printed therein, or adjacent thereto. This would allow a person to pick up the bottle 2 by the handle 118, and holding the handle 118 loosely they could then use the scale to measure the amount of liquid contained in the bottle 2. The bottle must be in the fully open configuration 124 to give an accurate measurement of its content's volume.

### Example 3: Disposal Test

As mentioned above, a disposable male urine bottle 2 should be easy to dispose. Accordingly, the inventors tested samples of the polyethylene laminated cardboard used to make the bottle 2 to ensure the material fully macerated in the standard macerator machine, which is found in most hospital wards. The material was deemed to macerate adequately enough to flow away in standard sewerage systems.

A second phase test was then undertaken which involved testing if the complete bottle 2 made from the polyethylene laminated cardboard would also fully macerate. Again, the material was deemed to macerate adequately enough to flow away in standard sewerage systems.

### Example 4: Water Proof Integrity Test

Any male urine bottle which can be used by the NHS has to have 100% leak integrity for a minimum of 5 hours. The inventors filled a bottle 2, made according to example 1, in the open configuration, with approximately 600ml of water. The inventors checked for leak evidence on an hourly basis. There was no evidence of leak failure after 24 hours, when the test was terminated.

### Example 5: Further Uses of the container

While the above examples have been concerned with using the container 2 as a disposable male urine container 2, it will be appreciated that it could also be used for other applications, such as a disposable container for using to hold liquid for drinking; as a disposable container for using to hold liquid for gardening purposes; as a disposable container for using to hold engineering liquids; as a disposable container for using to hold liquid for catering and kitchen applications; and as a disposable container for using to hold liquid for other purposes in the healthcare sector.

### Example 6: An alternative disposable flat-pack liquid container

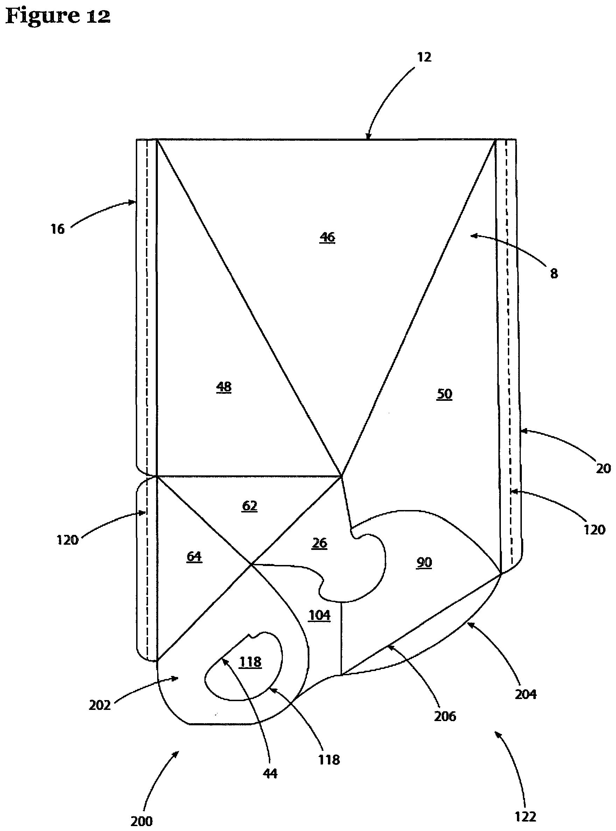

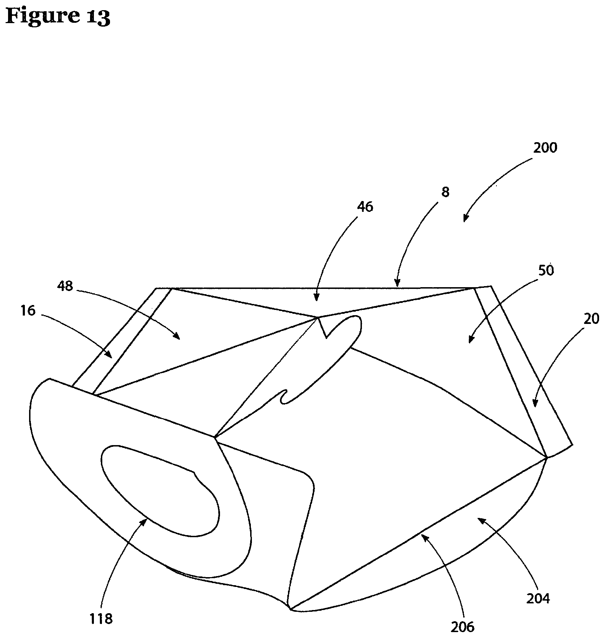

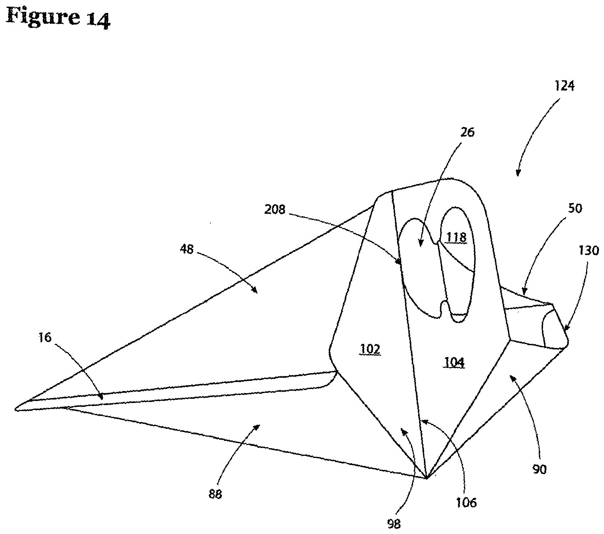

An alternative embodiment of the invention is shown in Figures 12 to 14. The bottle 200 shown in these Figures is similar to the bottle 2 shown in Figures 1 to 11 and described above.

However, the cut-out of bottle 200 does not comprise a distal flap 36. Instead, the cut-out defines a handle strengthening portion 202 in the first face 8. The handle strengthening portion 202 is disposed adjacent to the second isosceles triangle section 64 and defines the second aperture 40. The first face 8 is configured such that when the cut-out is folded along the central crease 6 to form the bottle 200, the second aperture 40 will lie directly above the first aperture 38, and define the handle 118.

Additionally, the cut-out 4 defines a flap portion 204 in the second face 10. The flap portion 204 is an elongate curved section disposed adjacent to the bottom right section 90. Additionally, the tenth volume crease 106 comprises a cut-out 208. The purpose for these are discussed below.

Finally, when the bottle 200 is manufactured, the folded cut-out is only heat-sealed along the left flanges 16 and the right flanges 20. No further heat-sealing is required, thereby simplifying the manufacturing process.

The bottle 200 may be converted to the open configuration 124 by applying opposing forces to the left and right flanges 16, 20, as explained above. While arrows 82, 84 are not shown in the Figures, it will be appreciated that while these are not essential but may be included in some embodiments if desired.

Similar to the bottle 2, once the bottle 200 is in the open configuration 124, the tab 26 can be folded through the aperture 38 in the handle 118. Due to the shape of the handle 118, the head 28 of the tab 26 is able to pass therethrough, and the neck 30 of the tab 26 can be placed in the indent 44. Furthermore, in this embodiment, the cut-out 208 is positioned to receive a distal end of the head 28 of the tab 26. Accordingly, the tab 26 is held in place at two points, thereby securely locking the bottle 200 into the open configuration 124.

The flap portion 204 can then be folded along crease 206 to define a bottom lip 130. It will be appreciated that the flap portion 204 can be folded prior to or after the bottle 200 has been converted to the open configuration 124. As explained previously, the folded crease is advantageous as it means the bottom lip 130, which will come into contact with a user's penis, is softened by the fold.

### Conclusion

As illustrated in examples 3 and 4 the bottle was found totally satisfactory and fit for purpose as a male urine bottle for use in an NHS hospital. As explained in example 1, the bottle 2 is flat when it comes off the production line, being the thickness of just two layers of polyethylene board. It is then a simple process for a nurse (or possibly a patient) to apply pressure to the bottle 2, where indicated by arrows 82, 84, and lock it into the open configuration 124 ready for use. The bottle 2 designed by the inventors will hold up to 1 litre of liquid, thereby removing the need for a range of different capacity products.

Inevitably much less space will be required for transportation to and storage in hospital wards, and many more units can be stored, thereby reducing transport costs. In addition, the complete supply chain will have a greatly reduced carbon footprint due to fewer lorry journeys, and greatly reduced demand on warehousing space and operations. In addition the high efficiency in transportation makes it possible to manufacture the bottle 2 further from the point of use, giving more opportunity to take advantage of possible cheaper production centres.

## Claims

1. A liquid container 2 comprising a rigid planar material defining first 8 and second faces 10 interconnected by at least two connecting edges 11, 13, each face 8, 10 having an inner surface and an outer surface, wherein the container is configured to be converted, upon application of substantially opposing forces to two sides of the container 2, from a planar configuration 122, in which the inner surface of the first face 8 is substantially adjacent to the inner surface of the second face 10, to an open configuration, in which the inner surfaces of the first and second faces 8, 10 are spaced apart and which, in combination with the at least two connecting edges 11, 13, define a volume therebetween for containing liquid, the container 2 comprises locking means configured to hold the container in the open configuration 124, and
wherein the locking means comprises a tab 26 disposed in the first face 8, and a first aperture 118 disposed in the second face 10, wherein the first aperture 118 is configured to receive the tab 26, thereby holding the container 2 in the open configuration 124, and **characterised in that** the at least two connecting edges 11, 13 comprise three connecting edges, and the three connecting edges comprise first, second and third side edges, wherein the third side edge intersects both the first and second side edges.

2. A liquid container 2 according to claim 1, wherein the planar material is coated or laminated with a waterproof material.

3. A liquid container 2 according to either claim 1 or claim 2, wherein the planar material comprises cardboard.

4. A liquid container 2 according to any preceding claim, wherein the planar material comprises bamboo, or biodegradable pulp, or corrugated board, or polyethylene-coated cardboard.

5. A liquid container 2 according to any preceding claim, wherein the first and second faces 8, 10 comprise one continuous piece of planar material, and the at least two connecting edges 11, 13 comprise a first folded edge and at least one further connecting edge, wherein the first folded edge is defined as a first fold in the planar material which separates the first face 8 from the second face 10.

6. A liquid container 2 according to any preceding claim, wherein the first face 8 comprises a first front edge, which is at least partially unconnected to the second face 10, and the second face 10 comprises a second front edge, which is at least partially unconnected to the first face 8, and when the container 2 is in the open configuration 124, the first and second front edges 11, 13 at least partially define an opening allowing access to the volume.

7. A liquid container 2 according to any preceding claim, wherein the tab 26 comprises a neck portion, which is connected to the remainder of the first face 8, and a head portion which is adjacent to the neck portion and comprises a maximum width which is greater than the width of the neck portion and the first aperture 118 comprises a substantially straight edge which defines a first indent or slot which is wider than the width of the neck of the tab 26 and narrower than the width of the head of the tab 26.

8. A liquid container 2 according to any preceding claim, wherein the second face 10 comprises a cut-out or slot configured to receive a distal end of the tab 26, and the tab 26 is sized to extend into the cut-out or slot when the container 2 is in the open configuration 124.

9. A liquid container 2 according to any preceding claim, wherein the container 2 comprises a strengthening section configured to strengthen the first aperture 118.

10. A liquid container 2 according to any preceding claim, wherein the height of the container 2 in the planar configuration is less than 10 mm, 9 mm, 8 mm, 7 mm, 6 mm, 5 mm, 4 mm or 3 mm.

11. A liquid container 2 according to any preceding claim, wherein the first and second faces 8, 10 each comprise a node, wherein when the container 2 is in the planar configuration, the first face node 54 is substantially adjacent to the second face node 94, and when the container 2 is in the open configuration 124, the first face node 54 is spaced apart from the second face node 94, and the distance between the nodes 54, 94 defines the maximum height of the container 2 in the open configuration 124.

12. A liquid container 2 according to claim 11, wherein the first and second face nodes 54, 94 are disposed closer to the second side edge than the first side edge, and the ratio of the distance that the first and second face nodes 54, 94 are disposed between the first side edge and the second side edge maybe between 120:60 and 91:89.

13. A liquid container 2 according to any preceding claim, wherein the first face 8 of the container 2 comprises a first folding section and the second face 10 comprises a second folding section, wherein when the container 2 is in the planar configuration 122, the inner surface of the first folding section is adjacent to at least a portion of the inner surface of the second folding section, and when the container 2 is in the open configuration 124, the first and second folding sections are adjacent to each other, and the inner surfaces of the first and second folding sections define an angle of greater than 45°.

14. A liquid container 2 according to claim 13, wherein when the container 2 is in the open configuration 124, the first and second folding sections are adjacent to each other, and the inner surfaces of the first and second folding sections are substantially planar.

15. A liquid container 2 according to either claim 13 or claim 14, wherein the first folding section comprises a right angled isosceles triangle.

## Patentansprüche

1. Flüssigkeitsbehälter 2, umfassend ein starres planares Material, das erste 8 und zweite Flächen 10 definiert, die durch mindestens zwei Verbindungskanten 11, 13 miteinander verbunden sind, wobei jede Fläche 8, 10 eine Innenfläche und eine Außenfläche aufweist, wobei der Behälter konfiguriert ist, um nach Anwendung von im Wesentlichen entgegengesetzten Kräften auf zwei Seiten des Behälters 2 aus einer planaren Konfiguration 122, in der die Innenfläche der ersten Fläche 8 im Wesentlichen an der Innenfläche der zweiten Fläche 10 angrenzt, in eine offene Konfiguration, in der die Innenflächen der ersten und zweiten Fläche 8, 10 beabstandet sind und die, in Kombination mit den mindestens zwei Verbindungskanten 11, 13 ein Volumen dazwischen zum Enthalten von Flüssigkeit definieren, konvertiert zu werden, wobei der Behälter 2 ein Verriegelungsmittel umfasst, das konfiguriert ist, um den Behälter in der offenen Konfiguration 124 zu halten, und
wobei das Verriegelungsmittel eine Lasche 26, die in der ersten Fläche 8 angeordnet ist, und eine erste Öffnung 118, die in der zweiten Fläche 10 angeordnet ist, umfasst, wobei die erste Öffnung 118 konfiguriert ist, um die Lasche 26 aufzunehmen, wodurch der Behälter 2 in der offenen Konfiguration 124 gehalten wird, und **dadurch gekennzeichnet, dass** die mindestens zwei Verbindungskanten 11, 13 drei Verbindungskanten umfassen, und die drei Verbindungskanten erste, zweite und dritte Seitenkanten umfassen, wobei die dritte Seitenkante sowohl die erste als auch die zweite Seitenkante kreuzt.

2. Flüssigkeitsbehälter 2 nach Anspruch 1, wobei das planare Material mit einem wasserdichten Material beschichtet oder laminiert ist.

3. Flüssigkeitsbehälter 2 nach Anspruch 1 oder Anspruch 2, wobei das planare Material Karton umfasst.

4. Flüssigkeitsbehälter 2 nach einem der vorangehenden Ansprüche, wobei das planare Material Bambus oder biologisch abbaubaren Zellstoff oder Wellpappe oder polyethylenbeschichteten Karton umfasst.

5. Flüssigkeitsbehälter 2 nach einem der vorangehenden Ansprüche, wobei die erste und zweite Fläche 8, 10 ein durchgängiges Stück aus planarem Material umfasst und die mindestens zwei Verbindungskanten 11, 13 eine erste gefaltete Kante und mindestens eine weitere Verbindungskante umfassen, wobei die erste gefaltete Kante als eine erste Falte im planaren Material definiert ist, die die erste Fläche 8 von der zweiten Fläche 10 trennt.

6. Flüssigkeitsbehälter 2 nach einem der vorangehenden Ansprüche, wobei die erste Fläche 8 eine erste Vorderkante umfasst, die mindestens teilweise mit der zweiten Fläche 10 unverbunden ist, und die zweite Fläche 10 eine zweite Vorderkante umfasst, die mindestens teilweise mit der ersten Fläche 8 unverbunden ist, und wenn der Behälter 2 in der offenen Konfiguration 124 ist, die erste und zweite Vorderkante 11, 13 mindestens teilweise eine Öffnung definieren, die Zugang zum Volumen gewährt.

7. Flüssigkeitsbehälter 2 nach einem der vorangehenden Ansprüche, wobei die Lasche 26 einen Halsteil, der mit dem Rest der ersten Fläche 8 verbunden ist, und einen Kopfteil umfasst, der an den Halsteil angrenzt und eine maximale Breite umfasst, die größer als die Breite des Halsteils ist, und die erste Öffnung 118 eine im Wesentlichen gerade Kante umfasst, die eine erste Vertiefung bzw. einen ersten Schlitz definiert, die bzw. der breiter als die Breite des Halses der Lasche 26 und schmaler als die Breite des Kopfs der Lasche 26 ist.

8. Flüssigkeitsbehälter 2 nach einem der vorangehenden Ansprüche, wobei die zweite Fläche 10 einen Ausschnitt bzw. Schlitz umfasst, der konfiguriert ist, um ein distales Ende der Lasche 26 aufzunehmen, und die Lasche 26 so größenbemessen ist, dass sie sich in den Ausschnitt bzw. Schlitz erstreckt, wenn der Behälter 2 in der offenen Konfiguration 124 ist.

9. Flüssigkeitsbehälter 2 nach einem der vorangehenden Ansprüche, wobei der Behälter 2 einen Stärkungabschnitt umfasst, der konfiguriert ist, um die erste Öffnung 118 zu stärken.

10. Flüssigkeitsbehälter 2 nach einem der vorangehenden Ansprüche, wobei die Höhe des Behälters 2 in der planaren Konfiguration kleiner als 10 mm, 9 mm, 8 mm, 7 mm, 6 mm, 5 mm, 4 mm oder 3 mm ist.

11. Flüssigkeitsbehälter 2 nach einem der vorangehenden Ansprüche, wobei die erste und zweite Fläche 8, 10 jeweils einen Knoten umfassen, wobei, wenn der Behälter 2 in der planaren Konfiguration ist, der Knoten der ersten Fläche 54 im Wesentlichen angrenzend an den Konten der zweiten Fläche 94 ist, und wenn der Behälter 2 in der offenen Konfiguration 124 ist, der Knoten der ersten Fläche 54 von dem Knoten der zweiten Fläche 94 beabstandet ist, und der Abstand zwischen den Knoten 54, 94 die maximale Höhe des Behälters 2 in der offenen Konfiguration 124 definiert.

12. Flüssigkeitsbehälter 2 nach Anspruch 11, wobei der Knoten der ersten und zweiten Fläche 54, 94 näher an der zweiten Seitenkante als an der ersten Seitenkante angeordnet sind und das Verhältnis des Abstands, in dem der Knoten der ersten und der zweiten Fläche 54, 94 zwischen der ersten Seitenkante und der zweiten Seitenkante angeordnet sind, zwischen 120:60 und 91:89 sein kann.

13. Flüssigkeitsbehälter 2 nach einem der vorangehenden Ansprüche, wobei die erste Fläche 8 des Behälters 2 einen ersten Faltabschnitt umfasst und die zweite Fläche 10 einen zweiten Faltabschnitt umfasst, wobei, wenn der Behälter 2 in der planaren Konfiguration 122 ist, die Innenfläche des ersten Faltabschnitts angrenzend an mindestens einen Teil der Innenfläche des zweiten Faltabschnitts ist, und wenn der Behälter 2 in der offenen Konfiguration 124 ist, der erste und zweite Faltabschnitt angrenzend aneinander sind und die Innenflächen des ersten und zweiten Faltabschnitts einen Winkel von nicht mehr als 45° definieren.

14. Flüssigkeitsbehälter 2 nach Anspruch 13, wobei, wenn der Behälter 2 in der offenen Konfiguration 124 ist, der erste und zweite Faltabschnitt angrenzend aneinander sind und die Innenfläche des ersten und zweiten Faltabschnitts im Wesentlichen planar sind.

15. Flüssigkeitsbehälter 2 nach Anspruch 13 oder Anspruch 14, wobei der erste Faltabschnitt ein rechtwinkliges gleichschenkliges Dreieck umfasst.

## Revendications

1. Récipient pour liquide 2 comprenant un matériau plan rigide définissant des première 8 et seconde 10 faces reliées par au moins deux bords de raccordement 11, 13, chaque face 8, 10 possédant une surface interne et une surface externe, ledit récipient étant conçu pour être converti, lors de l'application de forces sensiblement opposées sur deux côtés du récipient 2, depuis une configuration plane 122, dans laquelle la surface interne de la première face 8 est sensiblement adjacente à la surface interne de la seconde face 10, vers une configuration ouverte, dans laquelle les surfaces internes des première et seconde faces 8, 10 sont espacées et qui, en combinaison avec les au moins deux bords de raccordement 11, 13, définissent un volume entre elles pour contenir du liquide, le récipient 2 comprend un moyen de verrouillage conçu pour maintenir le récipient dans la configuration ouverte 124, et
ledit moyen de verrouillage comprenant une languette 26 disposée dans la première face 8 et une première ouverture 118 disposée dans la seconde face 10, ladite première ouverture 118 étant conçue pour recevoir la languette 26, maintenant ainsi le récipient 2 dans la configuration ouverte 124 et **caractérisé en ce que** les au moins deux bords de raccordement 11, 13 comprennent trois bords de raccordement et les trois bords de raccordement comprennent des premier, deuxième et troisième bords latéraux, le troisième bord latéral intersectant à la fois les premier et deuxième bords latéraux.

2. Récipient pour liquide 2 selon la revendication 1, ledit matériau plan étant revêtu ou laminé avec un matériau imperméable.

3. Récipient pour liquide 2 selon la revendication 1 ou 2, ledit matériau plan comprenant du carton.

4. Récipient pour liquide 2 selon l'une quelconque des revendications précédentes, ledit matériau plan comprenant du bambou, ou de la pâte biodégradable, ou du carton ondulé, ou du carton revêtu de polyéthylène.

5. Récipient pour liquide 2 selon l'une quelconque des revendications précédentes, lesdites première et seconde faces 8, 10 comprenant une pièce continue de matériau plan et lesdits au moins deux bords de raccordement 11, 13 comprenant un premier bord plié et au moins un bord de raccordement supplémentaire, ledit premier bord plié étant défini en tant que premier pli dans le matériau plan qui sépare la première face 8 de la seconde face 10.

6. Récipient pour liquide 2 selon l'une quelconque des revendications précédentes, ladite première face 8 comprenant un premier bord avant, qui est au moins partiellement non relié à la seconde face 10, et ladite seconde face 10 comprenant un second bord avant, qui est au moins partiellement non reliée à la première face 8, et lorsque le récipient 2 est dans la configuration ouverte 124, lesdits premier et second bords avant 11, 13 définissent au moins partiellement une ouverture permettant l'accès au volume.

7. Récipient pour liquide 2 selon l'une quelconque des revendications précédentes, ladite languette 26 comprenant une partie goulot qui est reliée au reste de la première face 8 et une partie tête qui est adjacente à la partie goulot et comprend une largeur maximale qui est supérieure à la largeur de la partie goulot et ladite première ouverture 118 comprenant un bord sensiblement droit qui définit une première indentation ou fente qui est plus large que la largeur du goulot de la languette 26 et plus étroite que la largeur de la tête de la languette 26.

8. Récipient pour liquide 2 selon l'une quelconque des revendications précédentes, ladite seconde face 10 comprenant une découpe ou une fente conçue pour recevoir une extrémité distale de la languette 26, et ladite languette 26 étant dimensionnée pour s'étendre dans la découpe ou la fente lorsque le récipient 2 est dans la configuration ouverte 124.

9. Récipient pour liquide 2 selon l'une quelconque des revendications précédentes, ledit récipient 2 comprenant une section de renforcement configurée pour renforcer la première ouverture 118.

10. Récipient pour liquide 2 selon l'une quelconque des revendications précédentes, ladite hauteur du récipient 2 dans la configuration plane étant inférieure à 10 mm, 9 mm, 8 mm, 7 mm, 6 mm, 5 mm, 4 mm ou 3 mm.

11. Récipient pour liquide 2 selon l'une quelconque des revendications précédentes, lesdites première et seconde faces 8, 10 comprenant chacune un nœud, lorsque le récipient 2 est dans la configuration plane, ledit premier nœud de face 54 étant sensiblement adjacent au second nœud de face 94, et lorsque le récipient 2 est dans la configuration ouverte 124, ledit premier nœud de face 54 étant espacé du second nœud de face 94 et ladite distance entre les nœuds 54, 94 définissant la hauteur maximale du récipient 2 dans la configuration ouverte 124.

12. Récipient pour liquide 2 selon la revendication 11, lesdits premier et second nœuds de face 54, 94 étant disposés plus près du deuxième bord latéral que du premier bord latéral, et ledit rapport de la distance à laquelle les premier et second nœuds de face 54, 94 sont disposés entre le premier bord latéral et le deuxième bord latéral, pouvant être compris entre 120:60 et 91:89.

13. Récipient pour liquide 2 selon l'une quelconque des revendications précédentes, ladite première face 8 du récipient 2 comprenant une première section de pliage et ladite seconde face 10 comprenant une seconde section de pliage, lorsque le récipient 2 est dans la configuration plane 122, ladite surface interne de la première section de pliage étant adjacente à au moins une partie de la surface interne de la seconde section de pliage, et lorsque le récipient 2 est dans la configuration ouverte 124, lesdites première et seconde sections de pliage étant adjacentes, et lesdites surfaces internes des première et seconde sections de pliage définissant un angle supérieur à 45°.

14. Récipient pour liquide 2 selon la revendication 13, lorsque le récipient 2 est dans la configuration ouverte 124, lesdites première et seconde sections de pliage étant adjacentes, et lesdites surfaces internes des première et seconde sections de pliage étant sensiblement planes.

15. Récipient pour liquide 2 selon la revendication 13 ou 14, ladite première section de pliage comprenant un triangle isocèle à angle droit.
